# EUROPEAN PATENT APPLICATION

(11) **EP 0 938 871 A2**
(43) Date of publication of application: **01.09.1999**
(21) Application number: 99301470.3
(22) Date of filing: 26.02.1999
(51) Int. Cl.: A61B 17/36

(54) **Surgical apparatus**

(30) Priority: 27.02.1998 US 31752
(71) Applicant: ECLIPSE SURGICAL TECHNOLOGIES, Inc., Sunnyvale, California 94089 (US)
(72) Inventor: Daniel, Steven A., Fremont, California 94539 (US); Harman, Stuart D., San Jose, California 95136 (US); Reynolds, Timothy C., Mountain View, California 94043 (US)
(74) Representative: Nicholls, Kathryn Margaret

(57) **Abstract**

A viewing and treatment apparatus for performing minimally invasive surgery through an opening in a patient's chest, the apparatus includes a visualization scope (100) and a working device such as a tissue ablation energy delivery device (510). The working device is encompassed within a working channel (134) that communicates with the distal end of the scope. In one embodiment, a bronchoscope is used whose catheter shafting (120) includes an introducer sleeve (102) at its distal section for providing stability during introduction to a patient's chest while stabilizing the treatment and visualization distal ends of the scope at a treatment site. This embodiment can include a needle piercing assembly (500) that cooperatively works with the working device (510) such as for drug delivery. In a second embodiment a rigid endoscope (200) with a viewing channel has an introducer member that is slidably disposed and detachable from the viewing channel and has a transparent convex distal tip (602) which provides non-articulation at the distal end. This introducer member can also be used in conjunction with the first embodiment where the catheter shaft requires non-articulation. Both types of visualization scopes include a working channel (134,606) in which the working device can translationally egress from the scope's distal end. Additionally, the rigid introducer member can be modified to include a movable transparent rotatable member for varying the direction which the working devices egresses the apparatus' distal end. The preferred surgical procedure is transmyocardial revascularization and the preferred working device is an optical fiber element that is translatable from the distal end of the apparatus during the procedure. The working device can optionally include a flexible piercing needle with an internal lumen which allows translation of a working device such as an optical fiber or act as a conduit for drug delivery.

## Description

This invention relates to surgical apparatus particularly for use in minimally invasive procedures. A preferred type of embodiment is a viewing surgical scope apparatus capable of introducing a visualization scope and a working device such as an energy delivery device in minimally invasive surgical procedures. In particular, the preferred procedure is transmyocardial revascularization "TMR" wherein the energy delivery device is an optical fiber element.

The human heart is a muscular dual pump that beats continuously throughout life sending blood to the lungs and the rest of the body. The interior of the heart consists of four distinct chambers. The septum, a thick central muscular wall, divides the cavity into right and left halves. On the right side, the upper half is known as the right atrium. Deoxygenated blood from the rest of the body arrives in the right atrium via the vena cava, the blood is pumped across a one-way valve known as the tricuspid valve into the lower portion known as the right ventricle. From there the blood circulates to the lungs through the pulmonary valve via the pulmonary artery where it is oxygenated by circulation through the alveoli of the lungs (not shown). The blood returns via the pulmonary veins to the left atrium and flows through a second valve, the mitral valve into the left ventricle where it is pumped via the aorta to the rest of the body.

Much of the heart consists of a special type of muscle called myocardium. The myocardium requires a constant supply of oxygen and nutrients to allow it to contract and pump blood throughout the vasculature. The inner surfaces of the chambers of the heart are lined with a smooth membrane, the endocardium, and the entire heart is enclosed in a tough, membranous bag known as the pericardial sac.

The pumping action of the heart has three main phases for each heart beat. *Diastole* is the resting phase during which the heart fills with blood: while deoxygenated blood is entering the right atrium, oxygenated blood is returned from the lungs to the left atrium. During *atrial systole*, the two atria contract simultaneously, squeezing the blood into the lower ventricles. Finally, during *ventricular systole* the ventricles contract to pump the deoxygenated blood into the pulmonary arteries and the oxygenated blood into the main aorta. When the heart is empty, *diastole* begins again. The electrical impulses which stimulate the heart to contract in this manner emanate from the heart's own pacemaker, the sinoatrial node. The heart rate is under the external control of the body's *autonomic nervous system*.

Though the heart supplies blood to all other parts of the body, the heart itself has relatively little communication with the oxygenated blood supply. Thus, the two coronary arteries, the left coronary artery and the right coronary artery, arise from the aorta and encircle the heart muscle on either side "like a crown" to supply the heart itself with blood.

Heart disorders are a common cause of death in developed countries. They also impair the quality of life of millions of people and restrict activity by causing pain, breathlessness, fatigue, fainting spells and anxiety. The major cause of heart disease in developed countries is impaired blood supply. The coronary arteries become narrowed due to *atherosclerosis* and part of the heart muscle is deprived of oxygen and other nutrients. The resulting *ischemia* or blockage can lead to *angina pectoris*; a pain in the chest, arms or jaw due to lack of oxygen to the hearts myocardium, or *infarction*; or tissue necrosis in myocardial tissue.

Techniques to supplement the flow of oxygenated blood directly from the left ventricle into the myocardial tissue have included needle acupuncture to create transmural channels (see below) and implantation of T-shaped tubes into the myocardium. Efforts to graft the omentum, parietal pericardium, or mediastinal fat to the surface of the heart had limited success. Others attempted to restore
arterial flow by implanting the left internal mammary artery into the myocardium.

Modernly, coronary artery blockage can be relieved in a number of ways. Drug therapy, including nitrates, beta-blockers, and peripheral vasodilator drugs (to dilate the arteries) or thrombolytic drugs (to dissolve clots) can be very effective. If drug treatment fails transluminal angioplasty is often indicated - the narrowed part of the artery, clogged with atherosclerotic plaque or other deposits, can be stretched apart by passing a balloon to the site and gently inflating it a certain degree. In the event drug therapy is ineffective or angioplasty is too risky (often introduction of a balloon in an occluded artery can cause portions of the atherosclerotic material to become dislodged which may cause a total blockage at a point downstream of the subject occlusion, thereby requiring emergency procedures, the procedure known as coronary artery bypass grafting (CABG) is the most common and successful major heart operation performed, with over 500,000 procedures done annually in America alone. The procedure takes at least two surgeons and can last up to five hours. First, the surgeon makes an incision down the center of the patient's chest and the heart is exposed by opening the pericardium. A length of vein is removed from another part of the body. The patient is subjected to cardiopulmonary bypass during the operation. The section of vein is first sewn to the aorta and then sewn onto a coronary artery at a place such that oxygenated blood can flow directly into the heart. The patient is then closed. Not only does the procedure require the installation of the heart-lung machine, a very risky procedure, but the sternum must be sawed through and the risk of infection is enhanced during the time the chest cavity is spread open.

Another method of improving myocardial blood supply is called *transmyocardial revascularization* (TMR), the creation of channels from the epicardial to the endocardial portions of the heart. The procedure uses needles to perform "myocardial acupuncture," that has been experimented with at least as early as the 1930s and used clinically since the 1960s, see Deckelbaum. L.I., Cardiovascular Applications of Laser Technology, *Lasers in Surgery and Medicine* 15:315-341 (1994). This technique has relieved ischemia by allowing blood to pass from the ventricle through the channels either directly into other vessels perforated by the channels or into myocardial sinusoids which connect to the myocardial microcirculation. This procedure has been likened to transforming the human heart into one resembling that of a reptile. In the reptile heart, perfusion occurs via communicating channels between the left ventricle and the coronary arteries. Frazier, O.H., Myocardial Revascularization with Laser - Preliminary Findings, *Circulation*, 1995; 92 [suppl II:II-58-II-65]. There is evidence of these communicating channels in the developing human embryo. In the human heart, myocardial microanatomy involves the presence of myocardial sinusoids. These sinusoidal communications vary in size and structure, but represent a network of direct arterial-luminal, arterial-arterial, arterial-venous, and venous-luminal connections. This vascular mesh forms an important source of myocardial blood supply in reptiles but its role in humans is not well understood.

Numerous TMR studies have been performed using lasers where channels are formed in the myocardium. In one study, 20-30 channels per square centimeter were formed into the left ventricular myocardium of dogs prior to occlusion of the arteries. LAD ligation was conducted on both the revascularized animals as well as a set of control animals. Results showed that animals having undergone TMR prior to LAD ligation acutely showed no evidence of ischemia or infarction in contrast to the control animals. After sacrifice of the animals post operatively between 4 weeks and 5 months, the laser-created channels could be demonstrated grossly and microscopically to be open and free of debris and scarring.

It is possible that the creation of laser channels in the myocardium may promote long-term changes that could augment myocardial blood flow such as by inducing angiogenesis in the region of the lazed (and thus damaged) myocardium. Support for this possibility is reported in histological evidence of probable new vessel formation adjacent to collagen occluded transmyocardial channels. In the case of myocardial acupuncture or boring, which mechanically displaces or removes tissue, acute thrombosis followed by organization and fibrosis of clots is the principal mechanism of channel closure. By contrast, histological evidence of patent, endothelium-lined tracts within the laser-created channels supports the assumption that the inside of the laser channels is or can become hemocompatible and that it resists occlusion caused by thrombo-activation and/or fibrosis.

U.S. patents that deal with TMR and myocardial revascularization include U.S. Patent 4,658,817 which teaches a method and apparatus for TMR using a laser. A surgical CO₂ laser includes a handpiece for directing a laser beam to a desired location. Mounted on the forward end of the handpiece is a hollow needle to be used in surgical applications where the needle perforated a portion of tissue to provide the laser beam direct access to distal tissue. U.S. Patent 5,125,926 teaches a heart-synchronized pulsed laser system for surgical TMR. This patent's system and method include a sensing device for synchronized firing of a laser during the contraction and expansion of a beating heart during a predetermined portion of the heartbeat cycle. This heart-synchronized pulsed laser system is important where the type of laser, the energy and pulse rate are potentially damaging to the beating heart or its action. Additionally, as the heart beats, the spatial relationship between the heart and the tip of the laser delivery probe may change so that the necessary power of the beam and the required position of the handpiece may be unpredictable. U.S. Patent 5,380,316 teaches of TMR performed by inserting a portion of an elongated flexible lasing apparatus into the chest cavity of a patient and lasing channels directly through the outer surface of the epicardium into the myocardium tissue. U.S. Patents 5,389,096 and 5,607,421 teach of myocardial revascularization that is performed by guiding an elongated flexible lasing apparatus into a patient's vasculature percutaneously such that the firing end of their respective lasing apparatus are adjacent the endocardium for lasing channels directly through the endocardium into myocardium tissue without perforating the heart's pericardium layer. None of
the above listed patents teach methods for performing myocardial revascularization using minimally invasive surgical techniques, nor do their respective systems include a device for visualizing areas of the heart during such a procedure.

Patent literature that deals with minimally invasive surgical procedures for myocardial revascularization includes PCT application WO 97/13468 and U.S. Patent 5,700,259 which teach of thoracoscopic myocardial revascularization devices using a CO₂ type laser based handpiece. U.S. Patents 5,685,857 teaches of a thoracoscopic cannula device. PCT Application WO 97/34540 teaches of video assisted thoracoscopic CO₂ type laser TMR surgical method for a thoracoscopic myocardial revascularization procedure.

Finally, viewing devices used in cardiac interventional procedures include U.S. Patents 4,784,133 and 4,976,710 which both teach of an angioscope/bronchoscope device that includes a flexible distal end with an inflatable balloon structure for viewing intravasculature structures. This device's flexible catheter includes a working channel for introducing a procedural device at the viewing/treatment distal end.

There is a need for an apparatus and method for performing myocardial revascularization from one or more minimally invasively formed penetrations and eliminating the need for open chest surgery by providing a viewing surgical scope allowing for single handed use during such a procedure.

In one aspect the invention provides a minimally invasive surgical apparatus comprising:
an elongated catheter having a proximal and a distal end and at least two working channels;
a visualization device within the first working channel;
a working device within the second working channel and translatable out of a distal end of the apparatus;
an introducer assembly coupled to the distal end of the catheter and having an essentially rigid proximal portion and a flexible distal portion;
a flexible cup member coupled to the flexible distal portion of the introducer assembly;
an actuation device for controlling operation and translation of the working device, operatively coupled to the catheter, said actuation device having a spring loaded triggering assembly.

In a second aspect the invention provides a minimally invasive surgical apparatus comprising:
an elongated catheter having a proximal and a distal end and at least two working channels;
a visualization device within the first working channel;
a piercing needle having a lumen within the second working channel and translatable out of the distal end of the apparatus;
a working device within the lumen of the piercing needle and translatable out of a distal end of the apparatus;
an introducer assembly coupled to the distal end of the catheter and having an essentially rigid proximal portion and a flexible distal portion; and
a flexible cup member coupled to the flexible distal portion of the introducer assembly.

In a third aspect the invention provides a minimally invasive surgical apparatus comprising:
a rigid tubular introducer having a proximal portion and an essentially transparent distal portion and a lumen configured to house a distal portion of a visualization device; and
at least a first working channel in combination with said introducer having proximal and distal portions and a terminus at the distal portion of the introducer, the first working channel configured to house an egressible working device for effectuating a minimally invasive surgical procedure, whereby said introducer enables viewing of the distal portion of the working channel.

The catheter may be flexible, rigid or semi-rigid.

The flexible distal portion of the introducer assembly is desirably deflectable.

The visualisation device may be rigid, largely rigid, or flexible.

An embodiment of the invention may be a surgical procedural apparatus for visualizing and treating tissue in a minimally invasive surgical procedure, the apparatus comprising:
a working device for effectuating the surgical procedure;
a visualization scope having a body member at a proximal end, an elongated flexible catheter attached to the body member, the catheter having at least two channels wherein a first channel provides optical viewing at a distal end of the apparatus and a second channel for introducing the working device to the apparatus, said second channel having a terminus at a distal portion of the catheter;
a deflection member for deflecting the distal portion of the catheter, said deflection member having an articulating handle member at a proximal end and pivotally mounted on the body member, the handle member includes an actuation member for controlling operation of the working device and translating a distal portion of the working device out from the terminus of the second working channel, the actuation member includes a triggering member that operates a spring loaded member that attaches to and advances a displacement member that attaches to a piercing needle member, the needle member has an internal lumen at the distal end of the working channel; and
an essentially rigid introducer member attached and encompassing a portion of the catheter juxtaposed to the distal end of the catheter, the length of the introducer member sufficient to effectuate penetration by the distal end of the working device at target tissue undergoing the procedure thereby stabilizing the distal end of the working device, the introducer member includes a handle member at a proximal end thereof for the placement of the introducer member within a patient, and the deflection member includes a flexible cup member to provide a working space for unobstructed visualization and treatment of target tissue.

An embodiment of the invention may be a surgical procedural apparatus for visualizing and treating tissue in a minimally invasive surgical procedure, the apparatus comprising:
a working device for effectuating the surgical procedure having a distal portion; a bronchoscope or other visualization scope having a distal and a proximal portion and a body member at the proximal portion, an elongated sleeve member for optical viewing at a distal portion of the apparatus attached to the body member; and
a rigid introducer and viewing assembly having a length sufficient to effectuate both penetration by the distal portion of the working device and treatment at a target tissue, the assembly including:
   a tubular member having a distal portion, said tubular member slidably encompasses the sleeve member,
   a working tube having a distal portion, said working tube defining a working channel having a distal portion, that attaches to the tubular member wherein the working device is configurably disposed therein and egressible at a distal portion of the assembly, and
   an inelastic and transparent distal tip member that encloses the distal portion of the tubular member wherein the distal portion of the working tube attaches to the tip member thereby enabling visualization of the distal portion of the working channel, whereby the introducer and viewing assembly stabilizes the distal portion of the apparatus and reduces active muscular tissue movement during the procedure; and wherein the visualization scope and sleeve member are preferably rigid and the distal portion of the working channel preferably has a terminus at the tip member. Preferably the distal portion of the visualization scope comprises an end surface, said end surface is essentially normal to an axial direction of the assembly thereby providing visualization in a forward direction, and the distal portion of the working channel includes at least one bend to direct the distal portion of the working device at a desired angle relative to the axial direction of the assembly, suitably essentially parallel or at an offset angle to the axial direction of the assembly. The distal portion of the visualization scope may comprise an end surface at an offset angle with respect to an axial direction of the assembly thereby providing visualization in a direction offset to the axial direction of the assembly. The distal portion of the working channel may include at least one bend to direct the distal portion of the working device at a desired angle relative to the axial direction of the assembly, suitably essentially parallel or at an offset angle with respect to the axial direction of the assembly.

An embodiment of the invention may be a surgical procedural apparatus for visualizing and treating tissue in a minimally invasive surgical procedure, the apparatus comprising:
a working device for effectuating the surgical procedure;
an endoscope having a body member at a proximal portion, an elongated rigid sleeve member attached to the body member, the sleeve member having an optical viewing channel for viewing a distal portion of the apparatus; and
a rigid introducer and viewing assembly having a length sufficient to effectuate both penetration by a distal portion of the working device and treatment at a target tissue, the assembly including:
   a tubular member that slidably encompasses the sleeve member,
   a working tube defining a working channel that attaches to the tubular member wherein the working means is configurably disposed therein and egressible at a distal end of the assembly, and
   an inelastic and transparent distal tip member that encloses a distal portion of the tubular member wherein a distal portion of the working tube attaches to the tip member thereby enabling visualization of a distal portion of the working channel,
      whereby the introducer and viewing assembly stabilizes the distal portion of the apparatus and reduces active muscular tissue movement during the procedure. Suitably the body member includes a pistol-type gripping handle, the working device includes an actuation device for controlling operation of the working device that is mounted to the handle, the actuation device includes an advancement member for translating the distal portion of the working device out from the terminus of the working channel through the tip member.

The invention may be used to carry out a method of performing a minimally invasive transmyocardial revascularization procedure on a heart comprising the steps of:
introducing through a minimally invasive penetration an apparatus having an integrated visualization device, piercing needle and working device, said piercing needle and working device independently translatable from a distal end of said apparatus, the distal end of the apparatus having an essentially rigid proximal portion and a flexible distal portion, and a flexible cup member coupled to the flexible distal portion of the introducer assembly;
viewing while advancing the piercing needle into the heart;
viewing while advancing said working device through the piercing needle;
effectuating the procedure with the working device. Generally, channels are formed in the heart thereby.

The invention may be used to carry out a method of performing a minimally invasive transmyocardial revascularization procedure on a heart comprising the steps of:
providing a minimally invasive apparatus comprising an elongated catheter with at least two lumens and a proximal and a distal end, said apparatus having a visualization device in a first lumen and a translatable working device in a second lumen, the distal end of said apparatus comprising an introducer assembly having an essentially rigid proximal portion and a flexible distal portion, and a flexible cup member coupled to the flexible distal portion of the introducer assembly, an actuation device coupled to the proximal end of the catheter for controlling operation and translation of the working device having a triggering member that operates a spring loaded member;
viewing the heart while advancing the working device into the heart;
viewing while effectuating the procedure with the working device; or a method of performing a minimally invasive surgical procedure in a body comprising the steps of:
   a) providing an apparatus comprised of a rigid introducer having a transparent distal portion configured to house a distal portion of a visualization device, and at least a first working channel in combination with said introducer, said working channel configured to house an egressible working device for effectuating the procedure;
   b) introducing the apparatus through a minimally invasive formed penetration;
   c) introducing the working device through the working channel; and
   d) viewing while effectuating the procedure.

A preferred embodiment of the present invention provides a method and apparatus for performing a minimally invasive surgical (MIS) procedure and in particular for the creation of a TMR channels in a heart wall. The surgical viewing scope apparatus comprises a visualization device such as a bronchoscope or endoscope in combination with a working device such as an optical fiber element or other energy delivery device which is introduced through a minimally invasive formed penetration of a patient's chest. The preferred use of the apparatus is to deliver sufficient energy to the heart wall to form a channel through at least a portion of the heart wall wherein the energy delivery device is introduced through a minimally invasive formed penetration in the patient's chest.

The first viewing surgical scope embodiment is an articulating bronchoscope with a mid-section introducer sleeve assembly for placement of the distal end of the viewing surgical scope through a patient's chest penetration. This embodiment of the viewing Surgical scope has an integrated working channel and an integrated handle member for providing both advancement of the working device and articulation of the distal end of the viewing surgical scope from which a working device can egress.

The second viewing surgical scope embodiment is a rigid endoscope with various designs of the working channel from which the working device can egress from the viewing surgical scope. This second embodiment includes a closed ended introducer sleeve member with a preferred convex viewing tip that can be pushed against the heart and allows viewing of a beating heart while performing the operation. This sleeve member acts as an introducer tubular member that also stops bleeding by applied pressure and can perform multiple operative procedures from the same chest wall penetration. This second embodiment can also include a pistol grip hand-piece with members for advancement and actuation of the working device. The introducer tubular member allows for quick disconnect and interchangeability for operating on both lateral, anterior and posterior sides of the heart from a single penetration in a patient's chest. The introducer tubular member is either a disposable or reusable member.

A method which may make use of the invention includes introducing a first viewing surgical device through a first minimally invasive penetration of a patient's chest. The first viewing surgical device includes a working channel. An energy delivery device is introduced through the working channel of the first viewing surgical device. Sufficient energy is delivered from the energy delivery device to the wall of the heart to form a channel through at least a portion of the wall. Another embodiment of the method includes forming first, second and third minimally invasive penetrations in a patient's chest. A first viewing scope device is introduced through the first minimally invasive penetration. The heart is prepared for channel formation by using tools introduced through the second and third minimally invasive penetrations. A second visualization device includes a working channel and is introduced through the third minimally invasive penetration. An energy delivery device is introduced through either the second minimally invasive penetration or the working channel of the second viewing surgical scope device. Sufficient energy from the energy delivery device is delivered to the heart wall and create a channel through at least a portion of the wall. The positioning of the visualization devices and the working tools can be interchanged between the first, second and third minimally invasively formed penetrations.

Preferred embodiments of the invention may enable one to provide one or more of the following:
(i) an apparatus and method using a minimally invasive surgical technique for TMR;
(ii) a method and apparatus for performing TMR through at least one minimally invasively formed penetration of a patient's chest;
(iii) a method and apparatus for TMR through two or more minimally invasively formed penetrations of a patient's chest;
(iv) a method and apparatus for TMR through a minimally invasively formed penetration in a patient's chest with an articulating viewing bronchoscope that includes at least one working channel, wherein multiple working channels could be incorporated for other procedural devices, such as a piercing needle for drug delivery at treatment sites;
(v) a method and apparatus for TMR through first and second minimally invasively formed penetrations in a patient's chest with a viewing surgical scope in the first penetration and a trocar configured to introduce working tools through the second penetration;
(vi) a method and apparatus for TMR by forming one or more minimally invasively formed penetrations and providing access to more than one region of the heart;
(vii) an apparatus for minimally invasive surgery (MIS) which is sufficiently rigid to support surrounding tissue, which allows channels to be created at angles to the apparatus' axis, e.g. normal to target tissue, or at an oblique angle to the target tissue site;
(viii) an apparatus for TMR which is atraumatic to surrounding tissue, minimizes bleeding, and reduces tissue movement at a target tissue site;
(ix) an apparatus having enhanced use and functional capabilities, such as a tissue piercing capability for added stability during the TMR procedure or drug delivery use;
(x) a method for a closed-chest formation of a channel in a wall of a heart in which an energy delivery device is introduced through a first minimally invasive penetration of a patient's chest. Sufficient energy is delivered from the energy delivery device to the wall of the heart to form a channel through at least a portion of the wall. In its simplest embodiment, a conventional pneumo-needle may be inserted through the chest wall and a laser waveguide inserted therethrough to form a channel, preferably using a viewing device to show the position of the advancing waveguide and the heart wall.

Numerous other advantages and features of the present invention will become readily apparent from the following detailed description of the invention and the embodiments thereof, from the claims and from the accompanying drawings.

### BRIEF DESCRIPTION OF THE FIGURES

FIG. 1A is a representative isometric view of a first embodiment of the viewing surgical scope apparatus of the present invention using articulated distal section members.
FIG. 1B is a section view of viewing surgical scope apparatus shown in FIG. 1A.
FIG. 2A is an isometric view of the distal end of the viewing surgical scope's introducer assembly shown in FIG. 1A.
FIG. 2B is a section view of FIG. 2A.
FIG. 2C is an exploded view of the distal end of the viewing surgical scope shown in FIG. 2A.
FIG. 3A is an isometric view of the proximal end of the viewing surgical scope apparatus shown in FIG. 1A.
FIG. 3B is an exploded view of FIG. 3A.
FIG. 4A is an isometric view of the optical fiber advancement and control handle assembly of the viewing surgical scope apparatus shown in FIG. 1A.
FIG. 4B is a section view of FIG. 4A.
FIG. 4C is an exploded view of FIG. 4A.
FIG. 5 is a representative side view of a piercing needle assembly used with the embodiments of the invention's viewing surgical scope apparatus.
FIGS. 6A, 6B, 6C & 6D are representative section views of viewing tubular assemblies that each have a clear distal tipped section with a working channel having various orientations at the clear distal tip.
FIG. 7 is a representative section view of a variation to the clear distal tip tubular member as shown in FIGs 6A-6D that has elements for controlling the working device's orientation at the viewing surgical scope's distal end.
FIGS. 8A is a second viewing surgical scope apparatus embodiment of the invention using a non-articulating viewing surgical scope that includes the clear distal tip tubular member shown in FIGs 6A-6D.
FIGS. 8B is a variation of the second viewing surgical scope embodiment of the invention using a non-articulating viewing surgical scope that includes the clear distal tip tubular member shown in FIGs 6A-6D where the handle uses a sliding advance mechanism for the working device.
FIG. 9 is a perspective view of a patient illustrating first, second and third minimally invasively formed penetrations formed in the patient's chest, such as used for access in TMR.
FIG. 10 is a perspective view of an interior of the patient's chest shown in FIG. 9.

It will be understood that the invention's preferred embodiments have many of the individual elements whose functional aspects are similar. Thus, it will be understood that structural elements having similar or identical functions may have like reference numerals associated therewith. The appended drawings illustrate only typical embodiments of this invention and are therefor not to be limiting of its scope, for the invention may admit to other equally effective embodiments.

### DETAILED DESCRIPTION

A minimally invasively formed penetration is a chest penetration that does not entail "open chest" surgery by gross spreading of the ribs or cutting through excessive ribs and/or the sternum. Minimally invasive surgery also involves formation of penetrations that may be performed intercostally or non-intercostally to access tissues and organs without large incision openings in a patient. Once devices have been introduced in this manner, treatments may be affected from within an organ outwards i.e. "inside-out," or in an "outside-in" manner. "Channels" refer to revascularization entries through the epicardium or myocardium and further includes entries that extend (i) through the endocardium from the epicardium; (ii) partially or fully through the myocardium; (iii) to form stimulation zones; or (iv) to form drug pockets. "Working devices" for treatment and diagnosis of affected coronary/vasculature tissue include devices configurable and extendable through a lumen within the viewing surgical scope's distal end such as: optical fiber elements capable of delivering laser energy with or without a piercing needle assembly at the distal end of the viewing surgical scope, drug delivery using a piercing needle assembly, RF tissue ablation devices, ultrasound devices, or mechanical coring devices.

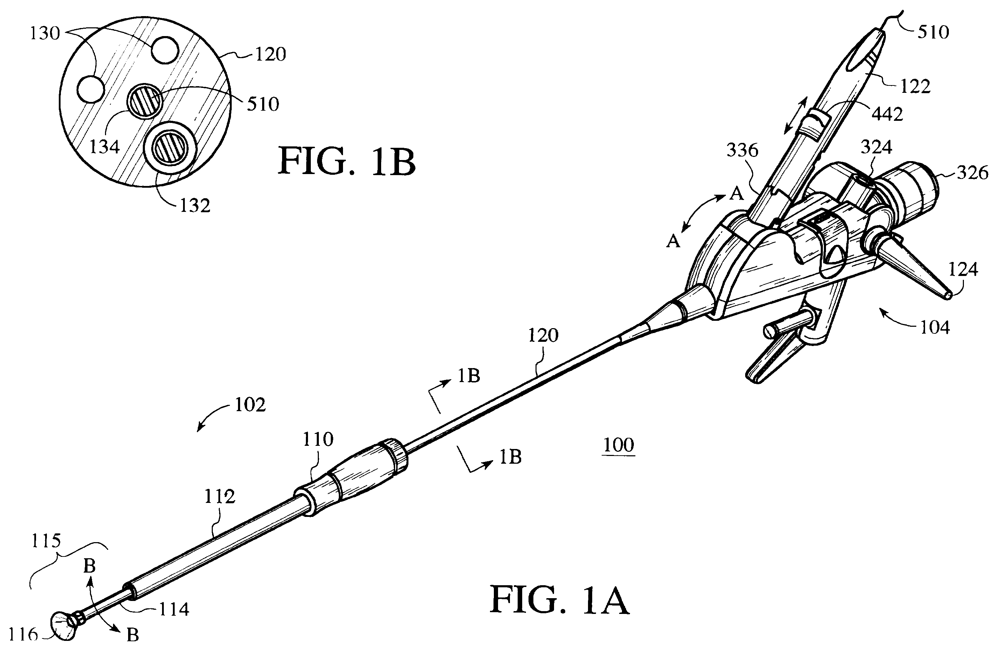

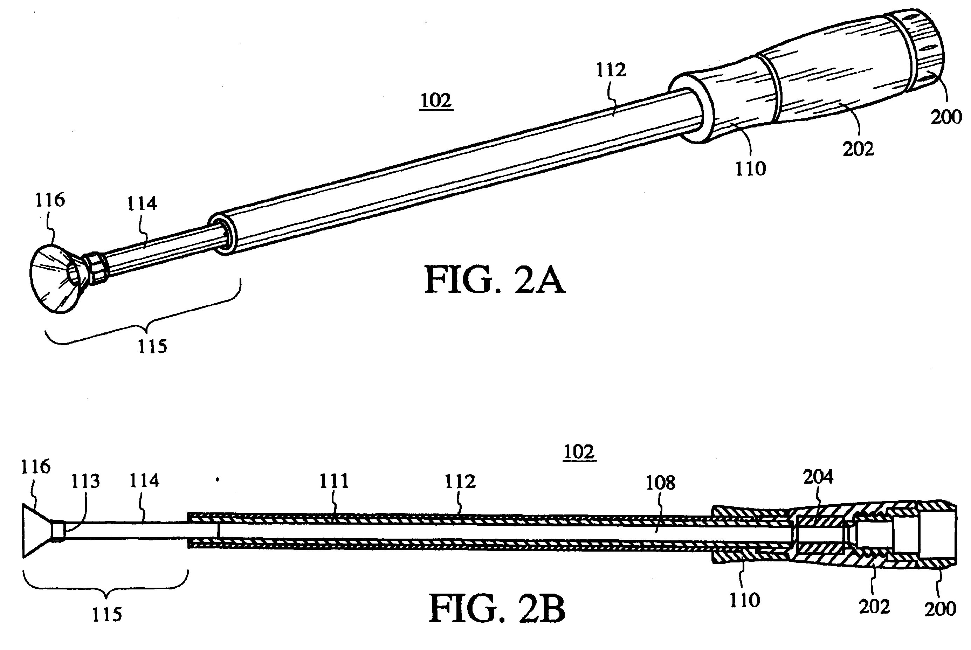

FIG.1A is a representative isometric view of the first embodiment of the invention's viewing surgical scope **100**. The viewing surgical scope **100** is an articulating bronchoscope with a distal end introducer assembly **102** and a main body assembly **104**. The introducer assembly **102** includes a handle portion **110** coupled to an essentially rigid tube **112**. Tube **112** surrounds a flexible member **114** with an attached suction cup **116** member. Catheter **120** couples to the main body assembly **104** and is either rigid, semi-rigid or flexible. A control handle **122** provides control of an optical fiber advancement member **442** of an optical fiber element **510** which transmits laser energy from a remote laser energy source. The bronchoscope's catheter **120** has multiple conduits which are accessed through the main body assembly **104** via multiple portal openings such as a fiber optic waveguide portal opening **124**. These conduits accomplish functions such as illumination, aspiration or irrigation of target tissue at the scope's distal end at suction cup member **116**. A hollow working channel is included within the catheter **120** for introducing implements such as a laser energy delivery optical fiber. The visualization scope shown can be a standard articulating bronchoscope or custom designed flexible endoscope made by Storz, Olympus or Pentax. The visualization scope's catheter **120** is within the bore **108** of the introducer assembly **102** shown in FIG. 2B.

FIG.1B is a section view at sectional line **1B-1B** of the viewing surgical scope **100** shown in FIG. 1A. The catheter **120** is a shaft of a bronchoscope with conduits **130** and visualization lumen with internal fiber **132** & working channel **134** with internal laser energy optical fiber element **510** extending the length of catheter **120** that communicates between the main body assembly **104** and the end at cup member **116**. In a typical configuration, one or more conduits **130** can be included within the catheter **120**. An eyepiece **326** shown in FIG.1A observes target tissue at the distal end of the viewing surgical scope **100** via the visualization lumen with internal imaging fiber **132**. Various types of ancillary viewing capabilities such as CCD monitoring can be attached at the eyepiece **326**. A translatable laser energy optical fiber element **510** is translatable and is disposed within the working channel **134** to deliver laser energy at the distal cup member **116** to form TMR channels in the heart.

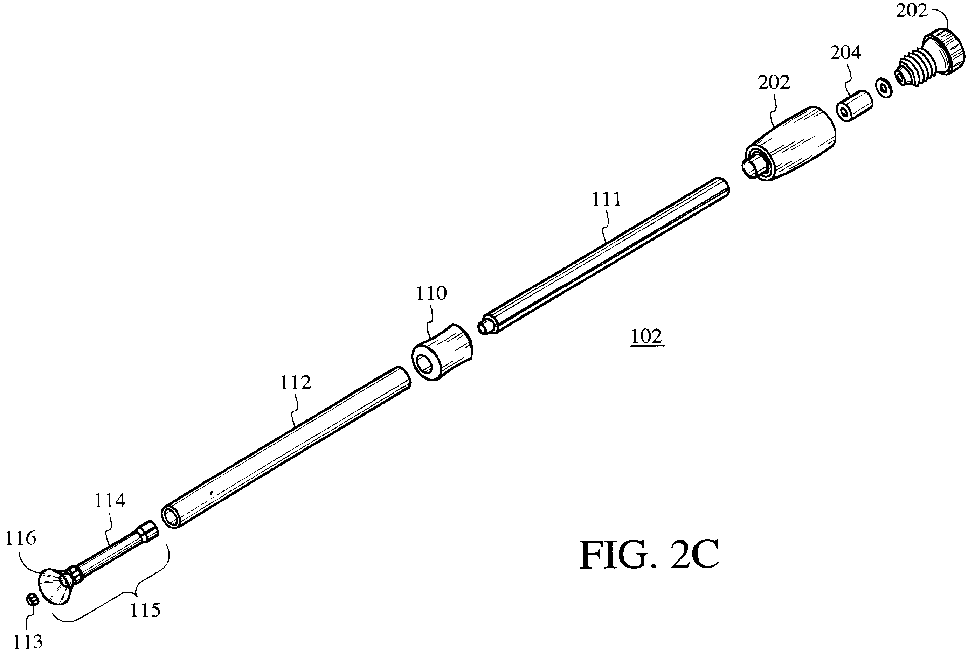

FIGs 2A, 2B & 2C show the introducer tubular assembly **102** of the viewing surgical scope **100** shown in FIG.1A. Handle member **110** couples, either by threaded member for quick uncoupling or permanently coupled thereto, to an essentially rigid tube **112**. A flexible tubular member **114** is attached either permanently to or slidably disposed within the tube **112**. Flexible tubular member **114** in turn is attached to the cup member **116**. The optional inner tube **111** is attached to the flexible tubular member **114** and the inner tube **111** is slidably disposed within the tube **112**. The inner tube **111** is made integral with the tube **112** when the tubular member **114** is permanently attached to tube **112**. Tube **111** when used attaches to the collet housing **202**, otherwise the tube **112** is attached thereto. The distal end of the catheter **120**, not shown in FIGs. 2A-2C, is disposed inside inner tube **111** and flexible tube **114** in the bore **108**. The catheter **120** is secured to the introducer tubular assembly **102** at a fixed location by manually tightening collet thumbscrew **200** into collet housing **202**, which compresses gripper **204**. A distal end lock ring **113** attaches the distal end of the catheter **120** to the cup member **116** as shown in FIG. 2C. The flexible tubular member **114** can be drawn into the tube **112** by making the cup member **116** smaller then shown such that when handle **110** can be decoupled from the collet housing **202** by twisting the handle **110** and then pushing handle member **110** with tube **112** towards the distal end of the scope **100**, the cup member **116** collapses and resides within the tube **112** thereby providing ease of scope **100** positioning through a minimally invasively formed penetration in a patient's chest so that entanglement with other instruments or internal body parts is minimized.

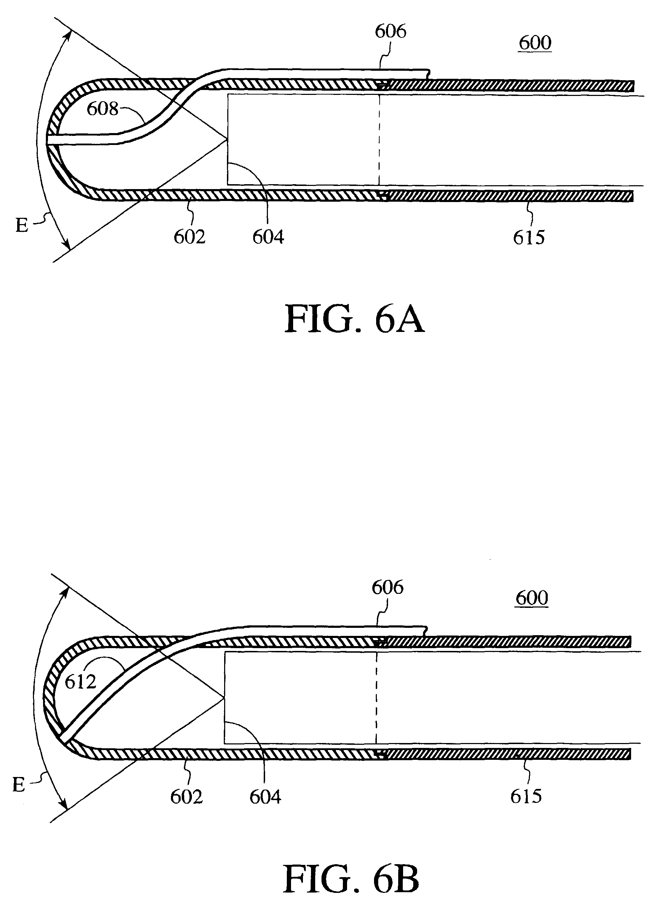

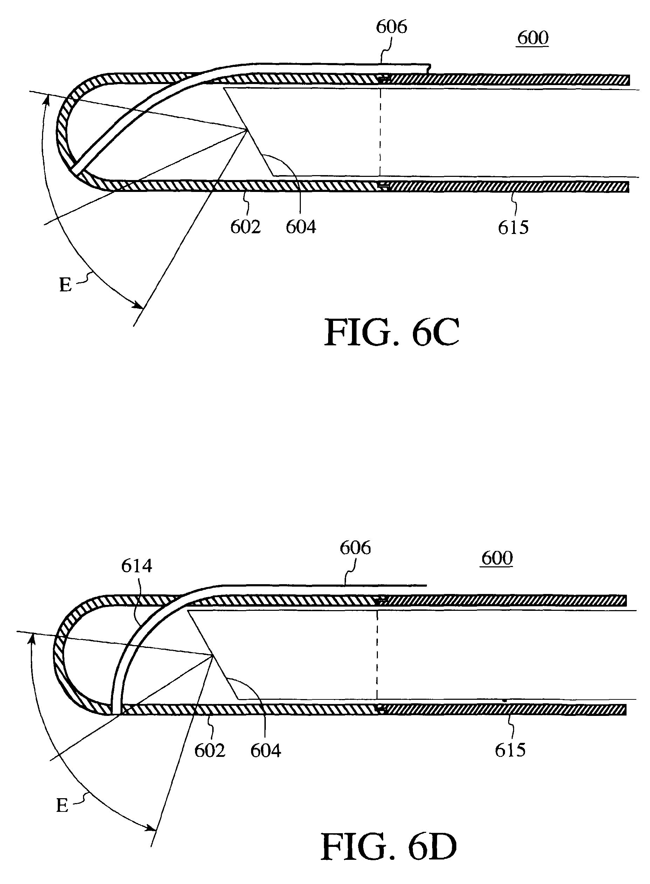

The flexible tubular member **114** and the suction cup member **116** form distal end assembly **115**. This articulating distal end assembly **115** is disconnectable and interchangeable with a essentially rigid non-articulating viewing tubular assembly **600** discussed below and shown in FIGs 6A-6D for a viewing surgical scope apparatus. The introducer tubular assembly **102** with catheter **120** is for insertion into a patient's chest through a minimally invasive penetration using the handle **110** for emplacement, see EP-A-0 858 779, which teaches of a trocar used for initially providing a chest wall penetration for introducing instruments into a chest cavity.

Catheter **120** comprises the elongated shafting of a bronchoscope or flexible endoscope tubing. The introducer tubular assembly **102** provides: a) stable support for emplacement within a patient's chest cavity and b) prevents unintended rotation and axial movement of the distal end of a working device such as the laser energy delivery optical fiber element **510**. The flexible tubular member **114** allows deflection at the distal end of the scope **100** by pivotal motions of the handle **122** which in turn causes a pivotal joint indicated by double arrow **A-A** in FIG.1A to push or pull a control wire (not shown) or an equivalent translational member communicating between the bronchoscope's proximal body assembly **104** and the distal end of the catheter **120**. Tip deflection mechanisms in bronchoscopes are well known in the art. The flexible tubular member **114** can be made of flexible silicon rubber or other elastic material with flexural characteristics for providing the necessary stability on a beating heart. Cup member **116** can optionally communicate with a vacuum source attached to the proximal body assembly **104** through port **324** via one of the internal conduits **130** to assist in heart wall attachment. Cup member **116** provides a broad surface which locks on the heart when evacuated for stability during the procedure. Cup member **116** keeps the optics clean and provides a protective shield for sharp tools which can scratch adjacent heart tissue. The cup member **116** can equivalently be a flange member with a flexible grooved annular surface for locking onto a heart surface with or without vacuum assist or be a flange member with a gripping textured surface that attaches to tissue during the procedure.

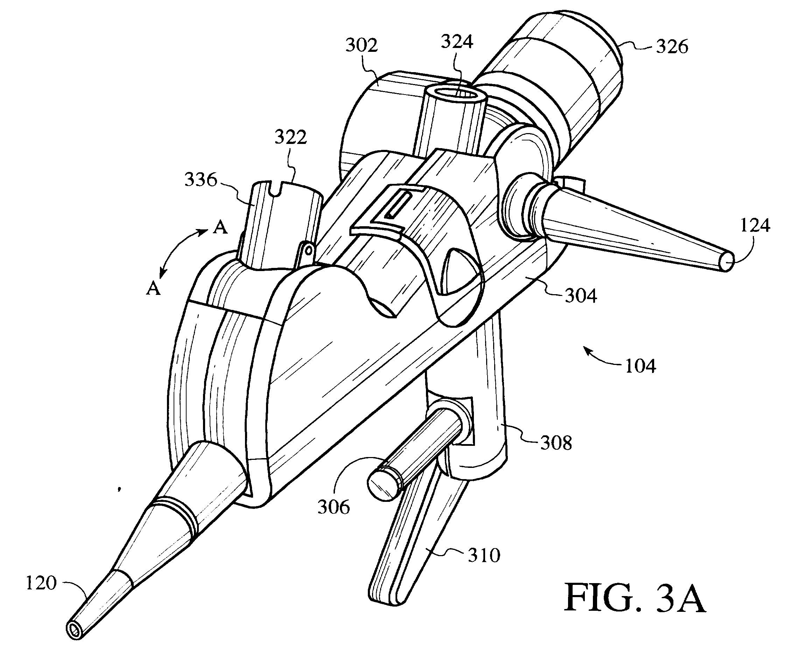

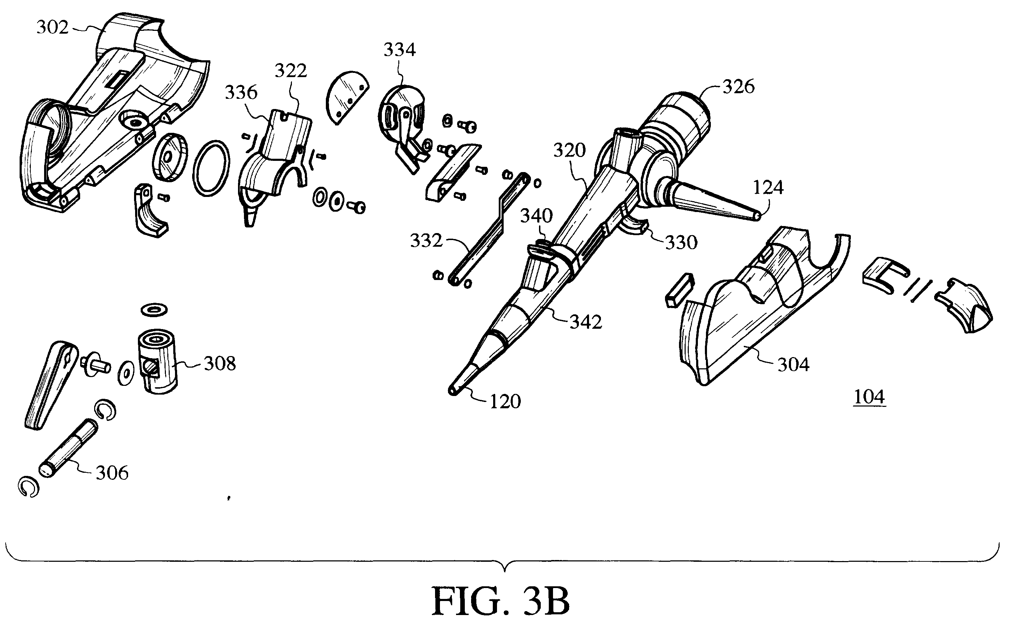

FIGs 3A & 3B are views of the main body assembly **104** as shown in FIG. 1A that can be mounted to the operating table or other structure using mounting shaft **306** that is attached to the body mount **308**. The body mount handle **310** allows manipulation of the main body assembly **104** when mounted to a fixture where the practitioner uses one hand to hold the introducer tube **112** at handle **110** and the other hand controls the handle **122** for optical fiber **510** translations and/or deflections of the distal end's cup member **116**. Main body assembly **104** in exploded view shown in FIG.3B has a right body housing **302** and a left housing body **304**. The right and left body housings **302** and **304** are configured as mating halves of an outer housing that encompass the proximal end of the visualization scope **342**, which is an articulating-type bronchoscope in this embodiment of the invention. The visualization scope **342** has at least two channels wherein a first working channel portal **322** communicates with the working channel **134** and the visualization portal through eyepiece **326**. A CCD-camera can optionally be used via the eyepiece **326**. Portal opening **124** typically provides illumination at target tissue sites at the distal end cup member **116**. Linkage **332** couples lever **330** via wheel linkage **334** to handle pivot member **336**. Pivoting of handle **122** shown by double headed arrow **A-A** in FIG. 1A results in articulation of the flexible member section **114** via control lever **330** action. The working channel port **322** optionally allows introducing procedural tools and instruments including but not limited to scissors, graspers, fiber optic tools, suture mechanisms without the pivot arm assembly as shown. Working channel port **322** with the handle **122** feature as discussed above substantially aligns with and allows free movement of the handle pivot member **336** through a ball joint socket design that couples to the port **340** on visualization scope **342**. Handle pivot member **336** allows translation of the working device such as an optical fiber element **510** therethrough.

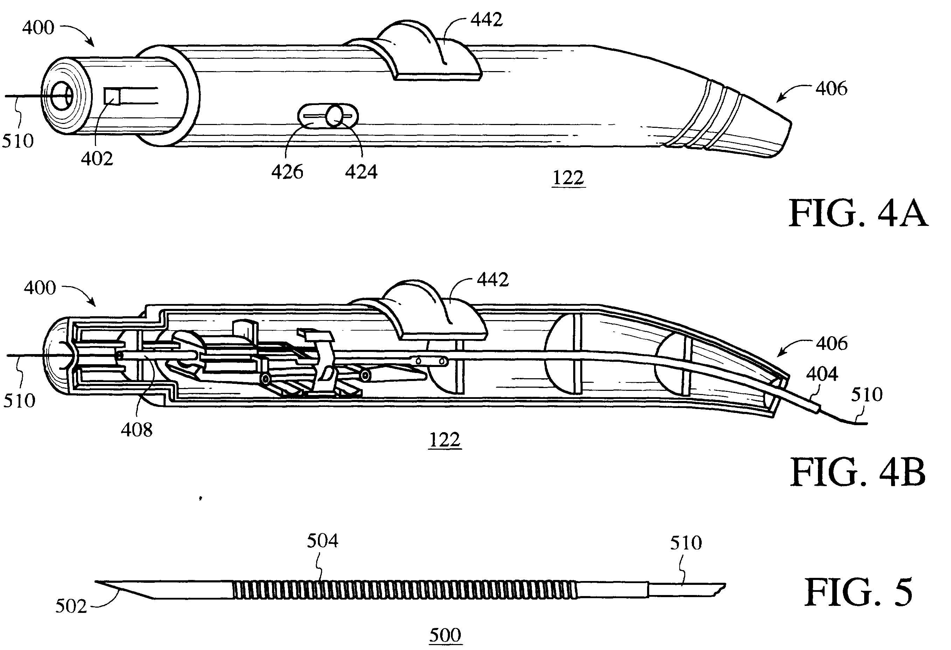

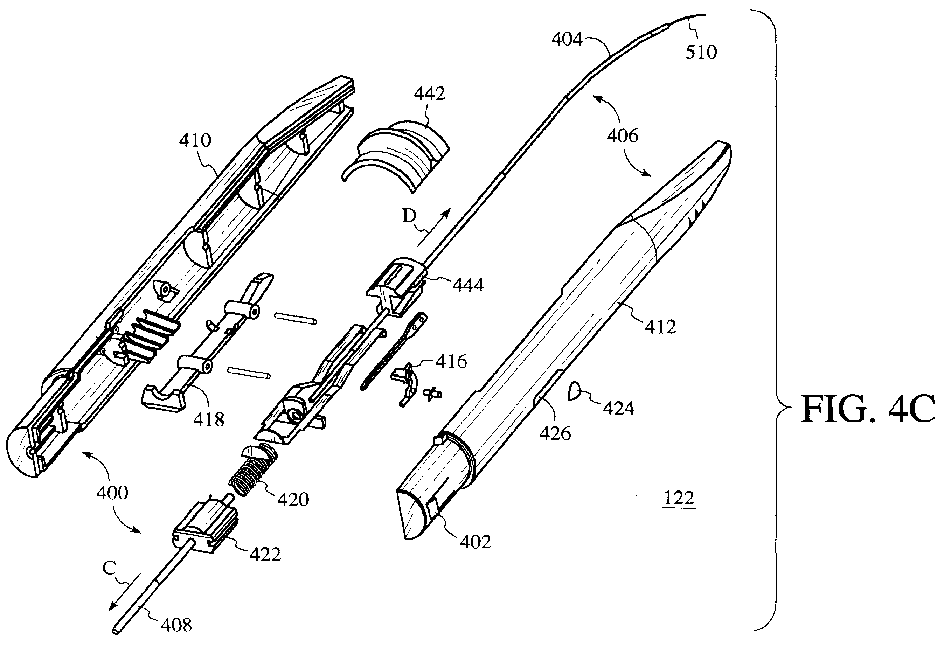

FIGs 4A & 4B are partial component views of the handle **122** with the optical fiber element thumb slider **442** shown in FIG.1A. FIG. 4B shows the handle **122** without the spring biasing element **420** and an interposed triggering/retraction leaf spring member and an internal slider **444** for clarity. FIG. 4C is an exploded view showing the internal components of the handle **122**. The thumb slider **442** advances and retracts the energy delivery device such as the optical fiber **510** independent of the triggered piercing needle member assembly as shown in FIG. 5. The handle **122** as discussed above moves in unison with handle pivot **336** shown in FIG. 1A thereby providing articulation of distal tip cup **116**. The practitioner's hand can control both the advancement of the optical fiber **510** and articulation of the distal tip cup member **116**. The distal end **400** of handle **122** is inserted into pivot handle member **336** and retained in place by locking member **402**. An end tube **404** sleeve enters the handle **122** at its proximal end **406** and another similar distal end tube **408** sleeve is disposed at the distal end **400** and extends to the distal end of the scope **100**. A mating right handle portion **410** and a left handle portion **412** are coupled together and enclose a needle piercing spring loaded drive assembly and energy delivery device advancement and control components. The optical fiber element **510** passes through the proximal and distal ends through tube **404** and a needle advance tube **408** which telescope with each other, the tube **404** is smaller than the tube **408** and the tube **404** attaches to the optical fiber element **510**, the tube **404** attaches internally to the internal slider **444** and the tube **404** slides within the tube **408**, thus allowing translation of the optical fiber **510** independent of the tube **408** movement. Movement of thumb slider **442** in direction **C** disengages a ratchet **416** in mechanical cooperation with a flexible latch **418** distal end locking member that disengages a piercing needle slider **422** resulting in needle advance spring **420** to push the needle slider **422** forward causing the needle advance tube **408** to move in direction **C** as well to advance the piercing needle distal end assembly **500** as shown in FIG. 5. Continued forward movement of thumb slider **442** advances the fiber optic element **510** through the needle advance end tube **408** which remains stationary. Movement of the thumb slide **442** is limited by fiber advance and depth stop button **424** slidably disposed within slot **426** by either a threaded compression or a biased detent member that cooperatively engages the slot **426** at predetermined positions. Finally, retraction of advance thumb slider **442** in the direction of arrow **D** causes the internal slider **444** to move rearwardly and causes the distal end of the triggering/retraction leaf spring member, which cooperatively slides within and engage internal slots in the slider **444**, to engage the distal end face of the slider **444** and pull the piercing slider **422** rearwardly as well, thus resetting and latching the needle slider **422** with spring **420** in relation to the latch **418** distal end face. The tube 408 is inserted into the working channel of the inventions viewing surgical scope apparatus.

FIG. 5 is a representative side view of the piercing needle assembly's distal end **500**. Piercing needle end portion **502** has a bevel cut end for piercing tissue and is coupled to a flexible section **504** which allows passage of the piercing needle distal end assembly **500** through a working channel with bending such as a flexible catheter or pre-shaped tubing. A fiber optic element **510** or other energy delivery device **510** passes through a lumen within piercing needle assembly **500** as shown in FIGS. 4A, 4B and 5. Moreover, the distal end needle assembly **500** can be a flexible drug delivery conduit and be a working device for the invention's viewing surgical scopes. Similarly, the distal end piercing needle assembly 500 can be replaced with a piercing optical fiber element as taught in U.S. Patent 5,703,985 entitled "Optical Fiber Device and Method for Laser Surgery Procedures," which is hereby incorporated by reference.

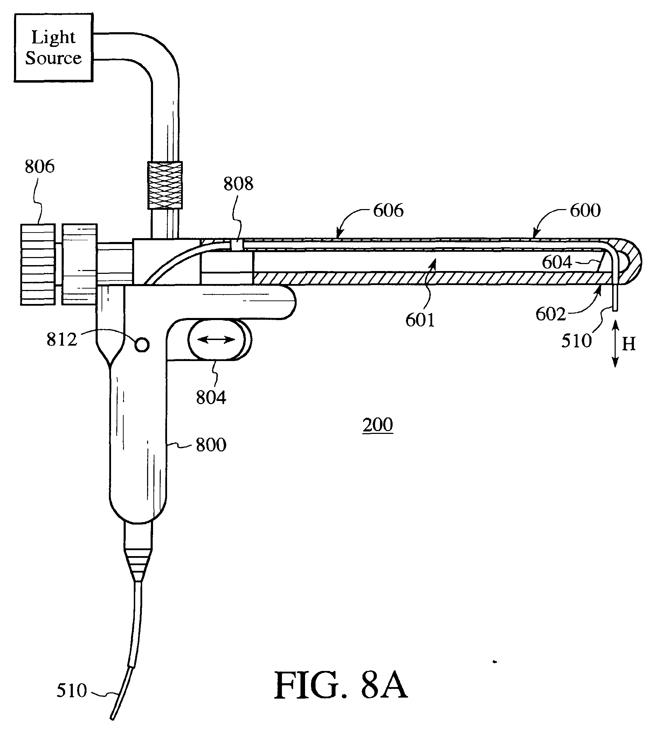

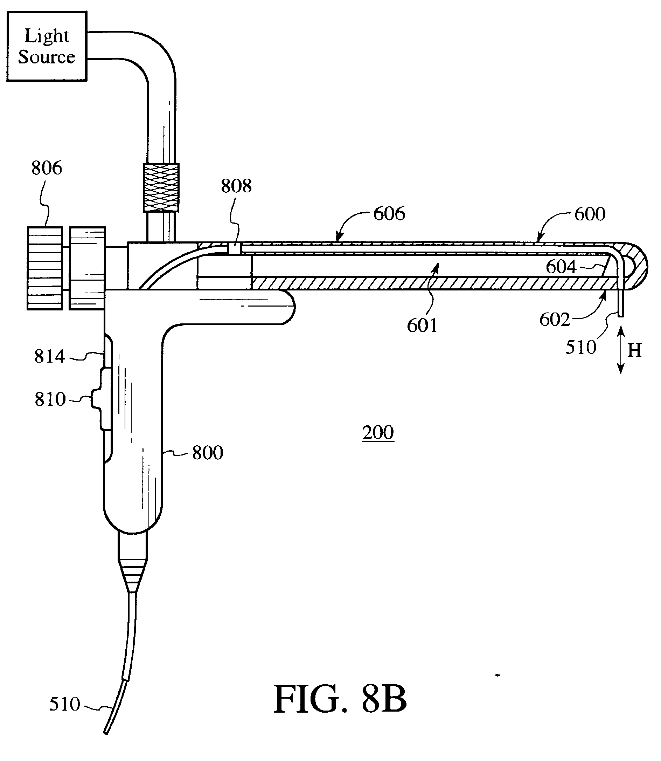

FIGS. 6A-D are representative section views of variations of a viewing tubular assembly **600**. The assembly 600 can be used with either a flexible or rigid endoscope. In particular, the assembly **600** as used with the viewing surgical scope **100** replaces the flexible distal end assembly **115** as shown in FIG. 1A; or alternatively and preferably used with a rigid shafted endoscope **200** discussed below and representatively shown in FIGs 8A & 8B. The viewing tubular assembly **600** includes an optically clear or transparent end tube cap **602** which fits over the visualization port distal end **604** of a scope's visualization shaft and has a working channel **606** (cut-off view). The distal ends **604** in FIGs 6A & 6B lie in planes essentially perpendicular to the central axis of the viewing tubular assembly **600** such that optics provide essentially direct forward visualization with a predetermined divergence viewing angle **E** as shown. The end port **604** shown in FIG. 6C is at a 30° angle with respect to the central axis of the viewing tubular assembly **600**. Distal end **604** can be varied such that the field of view is at an angle offset with respect to the central axis of viewing tubular assembly **600**. The viewing tubular assembly **600** replaces the components of flexible member **114** and cup member **116** in FIGs 2A-2C and cooperatively combines with the shaft member **112** and connectively interfaces representatively with the working channel **134** with appropriate tubing connectors with the working channel **606** shown in FIGs 6A-6D. The end cap **602** member is made from an acrylic or equivalent polycarbonate transparent material and coupled to a rigid tubular sleeve member **615**. Moreover, the assembly **600** can be a solid object made of the same material as the end cap **602** member. The distal end of the visualization scope **604** terminates near the transparent end cap **602**. The end cap **602** can made with desired optical light absorption/reflection characteristics. Furthermore, the shape of the end cap **602** can be conical, elliptical or include planar facets at various angles with respect to the viewing tubular assembly's **600** central axis. The end cap **602** is designed and made in accordance with required optical lens characteristics such as focus, divergence, convergence, directionability, collimation, polarization or diffusion.

The working channel **606** has various designs with differing bends that cooperatively are attached to the viewing tubular assembly **600**. The working channel **606** as shown is external to the assembly **600**, but can be incorporated into a lumen or be a structural tube either in the wall of the viewing tubular assembly **600** or conformably designed to fit within the inner wall surface of assembly **600** adjacent the distal end **604** of the visualization scope **342** or an end shaft of a rigid or flexible endoscope. The working channel **606** is shown attached to the external wall of assembly **600** in FIGS. 6A-6D. Viewing tubular assembly **600** functions to allow viewing of affected tissue while applying pressure to tissue for stopping bleeding and minimizing active tissue movement, e.g. a beating heart. The working channel **606** directs and protects the operative working device such as the optical fiber element **510**, a drug delivery needle or other energy delivery device that is controlled by handle **800** as shown in FIGS. 8A. The working channel **606** can be made of stainless steel, plastic or comparable material. In the preferred embodiment, the working channel **606** is clear to enable visualization of fiber movement. The working channel **606** in FIG. 6A has a curvature **608** such that the fiber or other working device is directed through the transparent end cap **602** in a direction essentially parallel with/or contiguous with respect to the central axis of the assembly **600**. The working channel **606** has a curvature **612** in FIG.6B which directs the working device through the transparent end cap **602** at approximately 45° with respect to the central axis of the assembly **600**. Likewise, the curvature **614** in the working channel **606** of FIG. 6C directs the working device through the transparent end cap **602** in a direction approximately 90° with respect to the central axis of the viewing tubular assembly **600**. Other orientations of working channel **606** and/or distal end bends in tube **606** can be used to direct the working device.

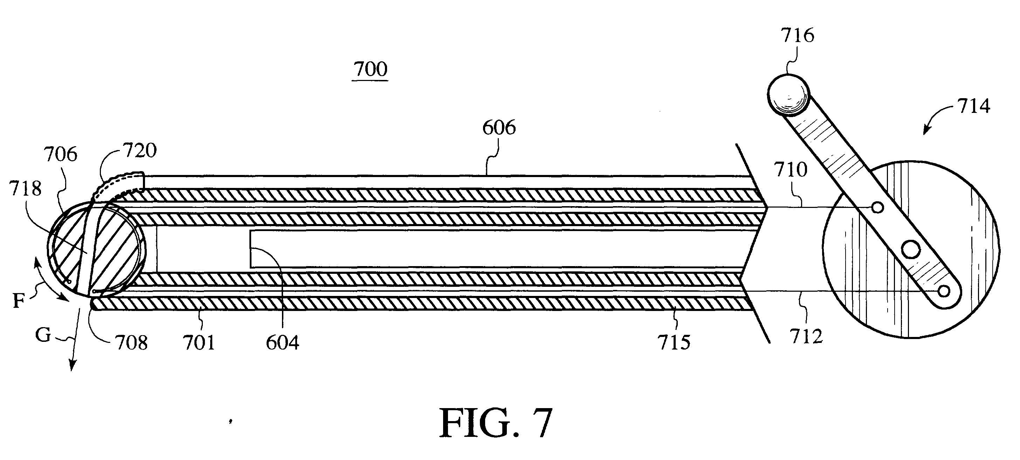

FIG. 7 is a representative section view of a variation of a movable distal ended optical ball viewing tubular assembly **700** that provides variable positioning of the working device such as the optical fiber **510** and can also be part of either viewing surgical scope **100** or **200** as discussed below. The optically transparent rotatable member **706**, which is either a ball or cylinder member, is at the distal end **708** and seats within a conformal shaped end tube **701** that allows free rotation of the rotatable member **706**. Upper steering wire **710** and a lower steering wire **712** are coupled to the rotatable member **706**. The steering wires **710** and **712** pass back to a proximal portion of the scope **100** or **200** to control mechanism **714**. The steering wires **710** and **712** are coupled to deflector knobs **716** for rotating the rotatable member **706** in a direction as shown by double headed arrow **F**. A guide channel **718** passes through the rotatable member **706**. A flexible coupling portion **720** extends between the guide channel **718** of the rotatable member **706** and the working channel **606**, thereby providing a path for directing the working device such as an optical fiber **510** therethrough. Flexible coupling portion **720** is a telescoping or an accordion-like interconnection allowing reorientation of the rotatable member **706** to direct the working device in a direction **G**. Tensioning steering wire **710** rotates the rotatable member **706** and re-directs the guide channel **718** in opposition to steering wire **712**. Additionally, more control wires can be includes to provide multiple degrees of rotation of the rotatable member **706** for greater controllability.

The articulating distal ended viewing tubular assembly **700** can replace the components of flexible member **114** and cup member **116**, i.e. assembly **115** in FIGs 2A-2C and cooperatively slides on shaft member **112**. The viewing tubular assembly **700** connectively interfaces at least with the conduits **130** and **134** with appropriate tubing channeling connectors and by appropriate internal control wire connections within the proximal end of sleeve member **715** and to appropriate connections in the flexible catheter shaft **120**. Moreover, the catheter **120** can be a stand alone viewing device whose distal end which representatively can be **604** in FIG. 7 and the work channel **606** would be tubing attached to the catheter 120 shafting. The viewing surgical scope **200** discussed below and shown as FIG. 8A and 8B would have a control member **714** on the handle **800** with connecting control wires **710** & **712**. The assembly **700** would encompass the rigid endoscope shafting **601** as discussed below.

The articulating assembly **700** of FIG.7 can have alternative designs such as an assembly comprising an internal mechanical deflecting linkage mechanism for changing the orientation of the egression angle of the working channel **606**. The transparent surface rotatable member **706** would be replaced with an essentially transparent cap member comparable to **602** with a flexible membrane to allow orientation displacement of the working channel **606** that is sealed within the membrane. Moreover, the deflecting linkage mechanism can be a light reflecting surface such that observations of tissue can be at offset angles with respect to the axial direction of the assembly tube **715** where the distal end of the visualization scope **604** has a normal surface with respect thereto.

FIG. 8**A** shows viewing surgical scope **200** with a handle assembly **800** using a finger trigger advance mechanism **804** and has the tubular viewing assembly **600**. The assembly **600** is non-articulating distal clear end cap **602** for visualizing and has a working channel **606** for directing the working device, e.g. an optical fiber **510** at a treatment site. The visualization scope is an endoscope whose distal end **604** is viewed through an eyepiece **806**. The distal end **604** of the endoscope can have different angular orientations as discussed above for a required distal end viewing field from the viewing tubular assembly **600**. The viewing surgical scope **200** for example can be a 10-mm sized rigid endoscope with a viewing tubular assembly **600** that has a 12 mm-O.D. A smaller 5-mm system endoscope, can also be used where the assembly **600** is about 10-12 mm O.D. that allows for additional space inside the assembly **600** for additional working channels **606** that allow for drug delivery, lighting etc. The handle assembly **800** is ergonomically designed for hand gripping. The handle assembly **800** includes a fiber advance mechanism using finger trigger **804** within the handle and alignment retaining members for attaching endoscope shafting **601** along with the viewing tubular assembly **600**. The viewing tubular assembly **600** is user removable for quick disconnect from the endoscope shafting **601** for quick interchange of tubular assemblies **600** with different working channel **606** egress angles for surgical procedures that occur at various aspects of the heart surface, such as the lateral, anterior, posterior or apexial walls when operating from a single chest penetration. The viewing tubular assembly **600** has a quick disconnect coupling member **808** for connections of the working channel **606** for quick interchangeability of the assembly **600**. Additionally, the articulated viewing tubular assembly **700** shown in FIG. 7 can be used with the necessary control features incorporated within the handle **800**. This feature allows access to lateral, anterior or posterior locations of an organ where a practitioner uses the same chest wall penetration.

Finger trigger **804** controls translatable movement of the working device, e.g. an optical fiber element **510** with or without a piercing needle distal end assembly **500** as shown in FIG. 5. The finger trigger **804** actuates mechanical or electrically movement of the working device from the distal end of the viewing tubular assembly **600** shown by the double arrow **H**, preferably using incremental control. Mechanisms for the advancement/retraction function include rack and pinion components, a stepper motor with appropriate control, pneumatic driven mechanisms with incremental stepping functional components.

Alternatively, the handle can include a slide member **810** as shown in FIG 8B which can include a mechanism comparable to that discussed above in FIGs 4A & 4B wherein a triggering mechanism advances a needle piercing member **500** and cooperatively works with the optical fiber **510** through an adjustable range, e.g.1.5- 2.5 cm. The slide member **810** can include detents for a user to sense rate of advancement. The advancement mechanism can also be geared to provide advancement at translation ratios other than 1:1. Retraction of the optical fiber **510** can be accomplished by reversing the trigger button **812** that cooperates with a reversing rack mechanism inside handle **800**. A stop setting member **814** can be used to position the optical fiber distal ending flush with the viewing tubular assembly's **600** outer surface. Alternatively, the mechanisms shown in FIGs 4A & 4B showing a slide controlled mechanism could be incorporated in handle **800** in lieu of the finger trigger **804**. An equivalent lever mechanism can be used in lieu of the finger trigger **804** which would include stops to limit optical fiber extension and retraction. In a TMR operation, the optical fiber element **510** would typically would be advanced in 1-mm increments.

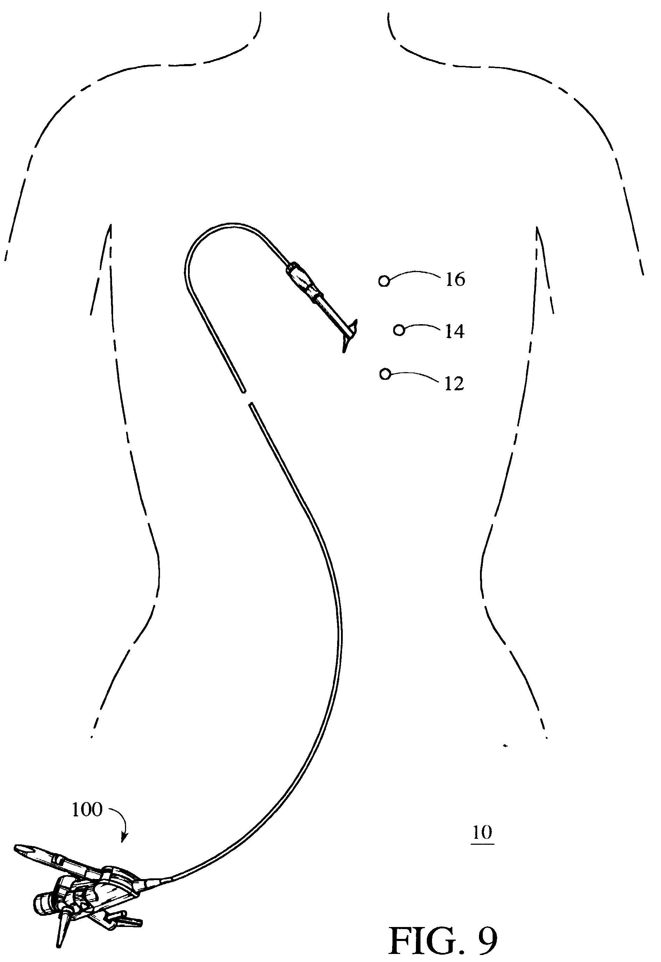

FIG. 9 shows a perspective view of a patient **10** with first, second and third minimally invasive formed penetrations **12**, **14** and **16** respectively. It will be appreciated that the exact location of penetrations **12**, **14** and **16** is not limited to those illustrated in FIG. 9. Additionally, from 1 to N+1 numbers of penetrations may be made. The patient is prepared for the procedure and is positioned similarly to that used for a left thoracotomy. The patient's left arm is draped. A conventional double lumen endotracheal tube is used to selectively deflate one side or the other of the lungs. Preferably the left lung is collapsed which allows access to the chest cavity in the vicinity of the left lung. The other lung remains inflated to provide oxygenation.

The distal portion of either viewing surgical scope **100** or **200** is positioned to reach a desired aspect of a ventricular wall. A plurality of different revascularization channels are formed in the heart. A distal portion of the energy delivery device or other working device can be positioned against tissue of the wall of the heart through which the channel is to be formed while transmitting energy from a remote energy source through the optical fiber element **510** or other energy delivery device. Additionally, the waveguide may be configured to pierce the epicardium, such as with a piercing needle as shown in FIG. 5, so that energy is or can be subsequently delivered to the myocardium. A revascularization channel can be formed through an epicardium into at least a portion of a myocardium or continue through the myocardium into all or only a portion of the endocardium.

In one method, penetration **12** is used for the introduction of either scope **100**, **200** or a separate rigid scope to provide global viewing capability of an internal chest area of interest. For standard TMR at the apex **20** region of the heart, a first penetration **12** can be formed in the intercostal spaces, for example the fourth to sixth intercostal space that is 10-12 mm in diameter. A slight cut is made and a thoracic trocar is advanced through the chest.

The scope **100**, **200** or separate rigid visualization scope is used to visualize the area, look for larger coronary vessels, to inspect the condition of the pericardium, and to check for adhesions. The shape of the heart as well as its position is visualized. Second penetration **14** is formed inferior to penetration **12** and can be formed just above the diaphragm and third penetration **16** is formed superior to penetration **12**. Penetrations **14** and **16** can be formed substantially the same way as penetration **12** is formed or may be cut downs only.

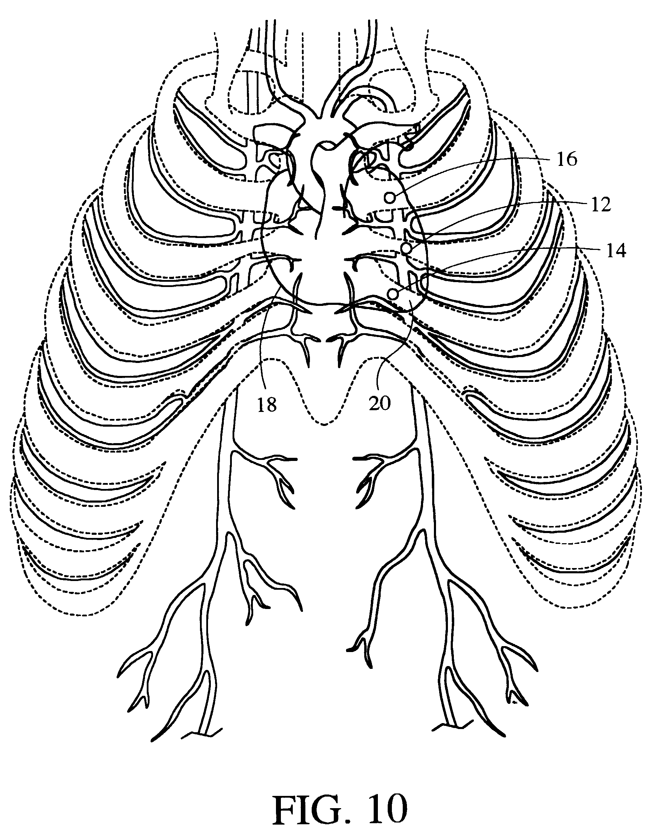

For initial procedures a pair of thoracoscopic graspers may be introduced through penetration **14**. Additional tools that can be introduced through penetration **14** include scissors. The pericardial sac **18** shown in FIG. 10, if intact, is grabbed and opened up using standard surgical techniques. The pericardial sac is pulled away from the heart and may be suspended. Unwanted adhesions are removed.

After the tools are removed from penetration **14**, either scope **100** or **200** with a working channel is introduced where the visualization scope, either a bronchoscope **342** or an endoscope can use a camera device attached to the eyepiece for viewing on a monitor. Additionally, additional viewing scope devices can be used during the procedure as inserted in the first penetration and the rigid scope can be inserted into second penetration **14** after the tools are removed from second penetration **14**.

Third penetration **16** is formed, a trocar introduced and a pair of forceps places an absorbing medium, including but not limited to a piece of gauze, through the third penetration **16**. Third penetration **16** is created initially to open the pericardial sac and subsequently may be used as a treatment port, for visualization or for safety reasons. In the event that a structure, such as a coronary artery is nicked and bleeding is initiated, direct pressure is applied by placing the gauze on the area through third penetration **16** to stop the bleeding. The gauze is also useful for manipulating the heart and applying slight pressure to TMR entrance sites to avoid excessive bleeding. When using the scope **200**, the tubular member assembly **600** stops bleeding when applied to areas undergoing treatment.

Either of the viewing surgical scopes **100** or **200** shown in FIGs 1A, 8A or 8B is initially positioned in penetration **14** and revascularization channels are created at the desired location, such as the apex **20**. Preferably the working device such as the energy delivery device is inserted through the working channel of either of the scopes **100** or **200** adapted for the procedure. The articulating-type scope **100** also may be initially positioned in penetration **12** or **16**. Once the desired number of revascularization channels are formed, either of the scopes **100** or **200** can be removed and positioned in any of the other penetrations. Graspers and needle holders, or other instruments, are introduced through one of the penetrations to stitch back the pericardial sac as required. A check is made to ensure that there is no bleeding, trocars are removed and the penetrations closed. It will be recognized that the procedure will vary, depending upon the condition of the heart and the site of the procedure.

In the preferred use of the present invention, the distal portion of the working device such as the energy delivery device is positioned to reach a desired aspect of a ventricular wall. A plurality of different revascularization channels are formed in the heart. A distal portion of the energy delivery device can be positioned against tissue of the wall of the heart through which the channel is to be formed while transmitting energy from a remote energy source through the energy delivery device.

Suitable working devices that can be inserted in the working channels of viewing surgical scopes **100** or **200** include energy delivery devices which include laser wave guides, RF electrodes, microwave cutters, ultrasound transmitters, mechanical coring devices or fluid jets. Each energy delivery device is configured to be coupled to an energy source including but not limited to RF, laser, microwave, ultrasound, mechanical coring, fluid jet, cryogenic fluid, chemical ablation and the like. The distal portion of the working device such as an energy delivery device can be positioned next to the heart wall while energy is delivered through the energy delivery device. Alternatively, the energy delivery device can deliver energy through a gaseous medium to the heart wall. The scopes **100** or **200** distal end can include a piercing obturator member for initial entry between the pericardial sac and the epicardium so that energy is delivered into the myocardium with minimal tissue destruction. A revascularization channel can be formed through an epicardium into at least a portion of a myocardium or continue through the myocardium into all or only a portion of the endocardium.

Other surgical procedures that scopes **100** or **200** could be used include gallbladder, laparoscopy or laparotomy, colectomy and other MIS operations that use other working devices for treatment of diseased tissue, such devices structurally configured for a working channel.

Unless defined otherwise, all technical and scientific terms used herein have the same meaning as commonly understood by one of ordinary skill in the art to which this invention belongs. Although any methods and materials similar or equivalent to those described can be used in the practice or testing of the present invention, the preferred methods and materials are now described.

While the principles of the invention have been made clear in illustrative embodiments, there will be immediately obvious to those skilled in the art many modifications of structure, arrangement, proportions, the elements, materials, and components used in the practice of the invention, and otherwise, which are particularly adapted to specific environments and operative requirements without departing from those principles. The appended claims are intended to cover and embrace any and all such modifications, with the limits only of the true purview, spirit and scope of the invention.

## Claims

1. A minimally invasive surgical apparatus comprising:
an elongated catheter having a proximal and a distal end and at least two working channels;
a visualization device within the first working channel;
a working device within the second working channel and translatable out of a distal end of the apparatus;
an introducer assembly coupled to the distal end of the catheter and having an essentially rigid proximal portion and a flexible distal portion;
a flexible cup member coupled to the flexible distal portion of the introducer assembly;
an actuation device for controlling operation and translation of the working device, operatively coupled to the catheter, said actuation device having a spring loaded triggering assembly.

2. A minimally invasive surgical apparatus comprising:
an elongated catheter having a proximal and a distal end and at least two working channels;
a visualization device within the first working channel;
a piercing needle having a lumen within the second working channel and translatable out of the distal end of the apparatus;
a working device within the lumen of the piercing needle and translatable out of a distal end of the apparatus;
an introducer assembly coupled to the distal end of the catheter and having an essentially rigid proximal portion and a flexible distal portion; and
a flexible cup member coupled to the flexible distal portion of the introducer assembly.

3. The apparatus of claim 2 further comprising:
an actuation device for controlling operation and translation of the working device, said actuation device having a triggering member that operates a spring loaded member operatively coupled to the piercing needle, whereby said working device is translatable independently of the piercing needle.

4. The apparatus of any preceding claim wherein the essentially rigid portion of the introducer assembly has a lumen, and the flexible distal portion of the introducer assembly and the flexible cup member are slidably disposed within said lumen.

5. A minimally invasive surgical apparatus comprising:
a rigid tubular introducer having a proximal portion and an essentially transparent distal portion and a lumen configured to house a distal portion of a visualization device; and
at least a first working channel in combination with said introducer having proximal and distal portions and a terminus at the distal portion of the introducer, the first working channel configured to house an egressible working device for effectuating a minimally invasive surgical procedure, whereby said introducer enables viewing of the distal portion of the working channel.

6. The apparatus of claim 5 wherein the proximal portion of the working channel is attached to an external surface of the introducer and the distal portion of the working channel is within the rigid introducer.

7. The apparatus of claim 5 wherein the proximal and distal portions of the working channel are within the rigid introducer.

8. The apparatus of claim 5 wherein the proximal portion of the working channel is within a wall of the rigid introducer.

9. The apparatus of claim 5 wherein the working channel is within the lumen of the rigid introducer.

10. The apparatus of any of claims 6-9 wherein the distal portion of the working channel is at an angle to the proximal portion of the working channel.

11. The apparatus of any of claims 5-10 further comprising a second working channel.

12. The apparatus of any preceding claim wherein the distal portion of the introducer has a conical shape.

13. The apparatus of any preceding claim wherein the introducer and/or at least the distal portion of the working channel is essentially transparent.

14. The apparatus of any preceding claim further comprising a shaft having proximal and distal portions and a first working channel coupled to the proximal portion of the introducer, said first working channel of the introducer being coupled to the first working channel of the shaft.

15. The apparatus of claim 14 further comprising a hand piece having a working device advancement mechanism and an actuation member, said hand piece being coupled to the proximal portion of the shaft.

16. The apparatus of any preceding claim wherein the working device comprises a laser energy delivery device and/or a piercing needle assembly and/or a coring device and/or a drug delivery device.

17. The apparatus of any preceding claim wherein the working device is at least one fiber optic.

18. The apparatus of claim 16 or 17 wherein the working device comprises a piercing needle assembly having a flexible portion.
